(19) Europäisches Patentamt
European Patent Office
Office européen des brevets

(11) **EP 4 530 632 A1**

(12) **EUROPEAN PATENT APPLICATION**

(43) Date of publication:
**02.04.2025 Bulletin 2025/14**

(21) Application number: **23210530.4**

(22) Date of filing: **17.11.2023**

(51) International Patent Classification (IPC):
**G01N 33/68** (2006.01)   **C07K 14/78** (2006.01)

(52) Cooperative Patent Classification (CPC):
**G01N 33/6848; C07K 14/78;** G01N 2030/8831;
G01N 2333/78

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.09.2023 CN 202311269285**

(71) Applicant: **Haleon US Holdings LLC
Wilmington, DE 19808 (US)**

(72) Inventors:
• **JU, Jessica
Suzhou, 215124 (CN)**
• **LI, Amy
Suzhou, 215124 (CN)**

(74) Representative: **Haleon Patent Department
5 The Heights, Wellington Way
Weybridge, Surrey KT13 0NY (GB)**

(54) **METHODS FOR DETECTING TYPE II COLLAGEN AMOUNT**

(57) The present invention provides a method for quantitative analysis of type II collagen, a kit for the method, and peptides.

EP 4 530 632 A1

**Description**

**FIELD**

The present invention relates to a method for quantitatively analyzing type II collagen.

**BACKGROUND**

Collagen is the most abundant protein in mammals. As the main component of extracellular matrix, it is widely present in tissues such as bone, tendon, cartilage, and skin. Collagen exhibits good biocompatibility, biodegradability, and weak immunogenicity. In recent years, with the rapid development of biomedicine and biotechnology, collagen has been widely used in medical devices, tissue engineering, regenerative medicine, food health, and the like. Therefore, establishing effective quantitative measurement methods not only contributes to the quality control of collagen-containing products, but also provides basic support for in-depth research on collagen related diseases.

Collagen is a triple helix structure formed by three $\alpha$-peptide chains. This special structure makes it difficult to be extracted and quantitatively analyzed. The most widely used method currently is the hydroxyproline (Hyp) method, which is based on the hydrolysis of collagen into free amino acids to measure the unique Hyp in collagen, and uses HPLC or photometric methods to determine its amount to calculate the amount of collagen. In addition, collagenase can be hydrolyzed into peptide fragments, and collagen can be quantified by measuring the amount of Gly Pro Hyp.

However, none of the above methods can identify the specific type of collagen, or quantify specific types of collagen. Therefore, there is an urgent need in this field to develop a fast and effective method for quantitative analysis of specific types of collagen, such as type II collagen.

**SUMMARY**

In an aspect, provided herein is a quantitative analysis method for type II collagen, including the following steps:

(1) Obtaining the solution of the sample;
(2) Using liquid chromatography-tandem mass spectrometry to measure the amount of type II collagen in the solution obtained from step (1), preferably using ultra high performance liquid chromatography-tandem mass spectrometry to measure the amount of type II collagen in the solution obtained from step (1).

In a specific embodiment, the above step (2) includes: (i) using liquid chromatography-tandem mass spectrometry to measure the amount of the standard peptide segment of type II collagen in the solution obtained from step (1); And (ii) convert the amount of the standard peptide segment measured in step (i) to the amount of type II collagen, wherein the standard peptide segment is selected from peptides with the sequence GIVGLOGQR or GSOGAQGLQGPR, preferably GSOGAQGLQGPR, where O represents hydroxyproline (Hyp).

In a specific embodiment, the amount of the standard peptide segment is measured using an internal standard method. When the standard peptide segment is a peptide with the sequence GSOGAQGLQGPR, the internal standard is preferably a peptide with the sequence GSOGA*QGLQGPR, where A* represents alanine with $^{13}$C, $^{15}$N, or D4 (deuterated) labeling, preferably alanine with $^{13}$C, $^{15}$N labeling.

In a specific embodiment, when the standard peptide segment is a peptide with the sequence GSOGAQGLQGPR and the internal standard is a peptide with the sequence GSOG (A-$^{13}$C$^{15}$N) QGLQGPR, the mass to charge ratio of the characteristic ions for quantifying the standard peptide segment is 571.5 and/or 627.4, and the mass to charge ratio of the characteristic ions of the internal standard is 573.5 and/or 627.4. Preferably, the amount of the standard peptide segment is determined based on the peak area of the characteristic ions with a mass to charge ratio of 627.4. When the standard peptide segment is a peptide with the sequence GSOGAQGLQGPR and the internal standard is a peptide with the sequence GSOG(D4-A)QGLQGPR, the mass to charge ratio of the characteristic ions of the internal standard is 573.5 and/or 887.9.

In a specific embodiment, the ion source of the mass spectrum in the liquid chromatography-tandem mass spectrometry is selected from the electric spray ion source (ESI), atmospheric pressure chemical ionization source (APCI), atmospheric pressure photoionization source (APPI) or matrix assisted laser desorption ionization source (MALDI), preferably the ESI positive ion mode; The quality analyzer is selected from quadrupole mass spectrometry, ion trap mass spectrometry, time of flight (TOF) mass spectrometry, or Fourier transform mass spectrometry; preferably, the mass spectrometry in the liquid chromatography-tandem mass spectrometry is a triple quadrupole mass spectrometry.

In a specific embodiment, the liquid chromatography is a reverse phase high-performance liquid chromatography, preferably using a C18 column, C8 column, or C4 column, and more preferably using a C18 column.

In a specific embodiment, gradient elution is performed using a mobile phase, which is a mixed system formed by formic acid aqueous solution and water-soluble non-proton organic solvent. The water-soluble non-proton organic solvent is preferably selected from acetonitrile, tetrahydrofuran, and/or dimethylformamide, more preferably acetonitrile.

In a specific embodiment, the mobile phase comprises mobile phase A and mobile phase B. The mobile phase A is a formic acid aqueous solution, and the mobile phase B is a mixed system of formic acid aqueous solution and water-soluble non-proton organic solvent.

In some embodiments, it also includes a step of cleaving proteins in the sample before detection, which involves enzymatic hydrolysis, chemical cleavage, and/or thermal cleavage, preferably enzymatic hydrolysis and/or chemical cleavage,

When the cleavage is enzymatic hydrolysis, it is preferable to use trypsin, chymotrypsin, thermophilic protease, pepsin, chymotrypsin, glutamic acid protease, lysine protease, aspartic acid protease, thrombin, and/or Staphylococcus aureus protease for enzymatic hydrolysis, and more preferably to use trypsin;

When the cleavage is chemical cleavage, it is preferable to use cyanide bromide, hydroxylamine hydrochloride, N-bromosuccinimide (NBS) and/or 3-bromo-3-methyl-2-(2-nitrophenylthio)-3H-indole (BNPS), and more preferably to use cyanide bromide;

Preferably, the sample is subjected to chemical cleavage and enzymatic hydrolysis, wherein the enzymatic hydrolysis is carried out simultaneously with, before, or after the chemical cleavage; More preferably, the enzymatic hydrolysis is performed after the chemical cleavage; Even more preferably, trypsin is used for enzymatic hydrolysis after chemical cleavage of the sample using cyanide bromide.

In some embodiments, it also includes a step of adding a reductant to the sample before detection, wherein said reductant preferably selected from dithiothreitol (DTT), β-mercaptoethanol, and/or tri(2-carboxyethyl) phosphine (TcEP), more preferably dithiothreitol (DTT); Preferably, the reductant is added to the sample after the chemical cleavage and before the enzymatic hydrolysis.

In some embodiments, the samples are tablets, capsules, granules, powders, small capsules, reconstitutable powders, dry powder inhalers, chewable agents, ointments, pastes, solutions, suspensions, injections, eye drops, liniments, collagen powder, or aerosols, preferably tablets.

In some embodiments, the type II collagen is derived from human, bovine, mouse, chicken, pig, or monkey sources, preferably from chicken cartilage, chicken joint cartilage, chicken chest cartilage, or pig joint cartilage.

On the other hand, provided herein is a kit comprising standard peptide segments and internal standards.

In a specific embodiment, the standard peptide segment is selected from peptides with a sequence GIVGLOGQR or GSOGAQGLQGPR, preferably GSOGAQGLQGPR, where O represents hydroxyproline (Hyp).

In a specific embodiment, when the standard peptide segment is a peptide with the sequence GSOGAQGLQGPR, the internal standard is preferably a peptide with the sequence GSOGA*QGLQGPR, where A* represents alanine with $^{13}$C, $^{15}$N, or D4 (deuterated) labeling, preferably alanine with $^{13}$C, $^{15}$N labeling.

In some embodiments, the kit also comprises a protein lysis agent, which is a protease and/or chemical cleavage agent, preferably selected from trypsin, chymotrypsin, thermophilic protease, pepsin, chymotrypsin, glutamic acid protease, lysine protease, aspartic acid protease, thrombin, and/or Staphylococcus aureus protease, The chemical cleavage agent is preferably selected from cyanide bromide, hydroxylamine hydrochloride, N-bromosuccinimide (NBS) and/or 3-bromo-3-methyl-2-(2-nitrophenylthio)-3H-indole (BNPS), and more preferably, the kit comprises trypsin and cyanide bromide.

In some embodiments, the kit also comprises a reductant, preferably selected from dithiothreitol (DTT), β-mercaptoethanol, and/or tri(2-carboxyethyl)phosphine (TcEP), more preferably dithiothreitol (DTT).

In some embodiments, the kit is used for measuring the amount of type II collagen, preferably for measuring the amount of type II collagen through liquid chromatography-tandem mass spectrometry.

On the other hand, provided herein is a use of peptides comprising a sequence selected from the following sequences for measuring type II collagen amount: GIVGLOGQR or GSOGAQGLQGPR, preferably GSOGAQGLQGPR, where O represents hydroxyproline (Hyp).

In a specific embodiment, the above peptide has a peptide sequence GSOGA*QGLQGPR, where A* represents alanine with $^{13}$C, $^{15}$N, or D4 (deuterated) labeling, preferably alanine with $^{13}$C, $^{15}$N labeling.

In a specific embodiment, the above peptides are used as internal standards.

**BRIEF DESCRIPTION OF DRAWINGS**

Figure 1 shows the standard curve established according to the method described in Example 2.

Figure 2 shows the STD3 chromatogram of the reference working solution.

Figure 3 shows the chromatogram of the working solution of Vitamins & Minerals type II collagen tablets.

## DETAILED DESCRIPTION

### Definitions

The following terms are intended to have the meanings presented below and are used to understand the description and scope of the present invention.

Unless otherwise defined, all technical and scientific terms used herein have the same meanings as those commonly understood by those skilled in the art to which the present invention belongs. The abbreviations used herein have their conventional meanings in the fields of chemistry and biology. The chemical structure and formula described herein are constructed based on standard rules of known chemical valences in the field of chemistry.

In the entire specification, where the composition and kit are described as having, including, or containing specific components, or where the process and method are described as having, including, or containing specific steps, it also include the case where the composition and kit of the present invention are mainly composed of or composed of the described components, and the case where the process and method of the present invention are mainly composed of or composed of the described processing steps.

In the present invention, where elements or components are referred to as being included in and/or selected from the list of described elements or components, it should be understood that elements or components can be any of the described elements or components, or elements or components can be selected from two or more of the described elements or components.

Furthermore, it should be understood that the elements and/or features of the composition or method described herein can be combined in multiple ways without departing from the spirit and scope of the present invention, regardless of whether being explicitly or impliedly stated herein. For example, when referring to a specific compound, the compound can be used in various embodiments of the composition of the present invention and/or in the methods of the present invention, unless otherwise understood from the context. In other words, in the present invention, embodiments have been described in a way that enables clear and concise writing and drawing of applications, but it is intended and will be understood that without departing from the teachings of the present invention and the present invention, embodiments can be combined or separated differently. For example, it should be understood that all features described and depicted herein can be applied to all aspects of the present invention described and depicted herein.

Unless the context is inappropriate, the articles "a" and "an" are used in the present invention to refer to one or more grammatical objects of the present invention (i.e., referring to at least one). By way of example, "an element" refers to one or more elements.

The term "and/or" is used in this invention to mean "and" or "or", unless otherwise specified.

It should be understood that the expression "at least one" includes each individual in the subject matter described after the expression, as well as various combinations of two or more of the subject matter described, unless otherwise understood from context and usage. The expression "and/or" related to three or more described objects should be understood to have the same meaning, unless otherwise understood from the context.

The terms "include", "includes", "including", "have", "has", "having", "contain", "contains", or "containing", including their grammatical equivalents, should generally be understood as open-ended and non-restrictive, such as not excluding additional undescribed elements or steps, unless otherwise specifically stated or understood from the context.

In the case where the term "about" is used before a quantitative value, the present invention also includes a specific quantitative value itself, unless otherwise specifically stated. As used herein, the term "about" refers to a variation of $\pm$ 10% from the nominal value, unless otherwise specified or understood from the context.

In various places in the present description, variables or parameters are disclosed in groups or ranges. Specifically, the present description intends to include each and each individual sub combination of such groups and scope members. For example, integers within the range of 0 to 40 are specifically intended to separately disclose 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, and 40, And integers within the range of 1 to 20 are specifically intended to disclose individually as 1, 2, 3, 4, 5, 6, 7, 8, 9, 10, 11, 12, 13, 14, 15, 16, 17, 18, 19, and 20.

The use of any and all examples or exemplary language (such as "such as" or "including") herein is only intended to better illustrate the present invention and does not limit its scope unless protection is required. The language in the present description should not be interpreted as indicating that any element not claimed to be protected is necessary for the practice of the present invention.

In general, the specified percentage of the composition is calculated by weight, unless otherwise specified. In addition, if the variable is not accompanied by a definition, the previous definition of the variable shall prevail.

The term "liquid chromatography-tandem mass spectrometry" used herein refers to an analytical technique in which liquid chromatography is used for separating the analyte from the interfering substance, and mass spectrometry is used for detection. After the sample is injected, it is first carried by the mobile phase and enters the chromatographic column. After separation by the chromatographic column, it enters the mass spectrometry for detection. Mass spectrometry detects the mass to charge ratio (m/z) of the tested substance, which is converted into gas phase ions at the ion source and enters the mass spectrometry.

The term "internal standard method" used herein refers to a quantitative method in which a certain weight of pure substance is added as an internal standard to a certain amount of sample mixture to be analyzed, and then the sample containing the internal standard is subjected to chromatographic analysis. The peak area (or peak height) and relative correction factor of the internal standard and the analyzed component are measured, and the percentage amount of the analyzed component in the sample is calculated according to a formula.

The term "protease" used herein refers to an enzyme that catalyzes the hydrolysis of peptides or proteins.

The term "reductant" used herein refers to a substance that prevents substances such as proteins from losing their activity due to oxidation.

The amino acid sequences used herein contain standard single letter or three letter codes for 20 naturally occurring amino acids. The code covers the isotope labeled forms of corresponding amino acids and the forms that are not labeled by isotopes.

## Collagen

Collagen is a biomacromolecule protein synthesized by animal cells, widely present in animals and the most abundant protein in mammals. Collagen, as the main component of extracellular matrix, is widely present in tissues such as bone, tendon, cartilage, and skin, and has a significant impact on the normal function and damage repair of cells, tissues, and even organs. In recent years, with the rapid development of biomedicine and biotechnology, collagen has been widely used in fields such as medical devices, tissue engineering, and regenerative medicine.

At present, at least 49 different collagen peptide chains have been discovered, and the number of collagen types that can be formed has increased to 28. According to the characteristics of collagen fibrils, collagen can be categorized into two categories: fibrillar collagen with periodic stripes and non-fibrillar collagen without periodic stripes. Among them, fibrillar collagen accounts for about 90% of the total collagen protein, including type I, II, III, V, VI, XXIV, and XXVII collagen, while the other types are non-fibrillar collagen. According to its distribution in the body, collagen can be further categorized into interstitial collagen, basement membrane collagen, and extracellular collagen. Due to the abundance of collagen in the intercellular matrix, interstitial collagen accounts for the vast majority of the entire body's collagen, including type I, II, and III collagen molecules.

A typical collagen molecule is a long and strong right-handed superhelix structure formed by the interlocking and winding of three left-handed helical $\alpha$-peptide chains of polyproline type II, known as the "triple helix domain". The primary structural analysis shows that the biggest characteristic of the triple helix domain is the Gly-X-Y periodic repetitive arrangement of amino acids, where the X position is usually proline (Pro), the Y position is usually hydroxyproline (Hyp) and hydroxylysine (Hyl), and the latter two amino acids are rare in other proteins. All types of collagen have this repetitive sequence, but in the triple helix domain of non-fibrillar collagen, this repetitive sequence is disrupted at certain specific locations.

## Type II Collagen

Type II collagen is composed of three $\alpha$1 peptide chains, mainly distributed in cartilage and vitreous body. Mature type II collagen does not contain tryptophan and differs in amino acid composition from the other two collagen types I and III. For example, the amount of hydroxylysine in type II collagen is 2-4 times that of type I and III collagen, and most hydroxylysine is glycosylated.

The fiber diameter formed by the self-assembly of type II collagen protein is relatively small, forming a fine network structure in cartilage. It is the most important organic component in the cartilage matrix and is a characteristic protein of cartilage tissue. It closely binds with polysaccharides, making cartilage flexible, capable of absorbing shocks and withstanding loads. Most physiological characteristics of cartilage depend on the integrity of the type II collagen network.

Research has shown that type II collagen has good biocompatibility and low antigenicity. Appropriate doses of type II collagen can prevent or alleviate symptoms of osteoarthritis and rheumatoid arthritis.

## Methods for measuring the amount of type II collagen

Provided herein is a detection method for type II collagen, comprising the following steps:

(1) obtaining the solution of the sample;
(2) measuring the amount of type II collagen in the solution obtained from step (1) using liquid chromatography-tandem mass spectrometry.

In some embodiments, the above step (2) comprises: (i) measuring the amount of the standard peptide segment of type II collagen in the solution obtained from step (1) using liquid chromatography-tandem mass spectrometry; and (ii) converting the amount of the standard peptide segment measured in step (i) to the amount of type II collagen, wherein the standard peptide segment is selected from peptides with sequences GIVGLOGQR or GSOGAQGLQGPR, where O represents hydroxyproline (Hyp). In a specific embodiment, the standard peptide segment is GSOGAQGLQGPR.

In a specific embodiment, the amount of the standard peptide segment is measured using an internal standard method. When the standard peptide segment is a peptide with the sequence GSOGAQGLQGPR, the internal standard is preferably a peptide with the sequence GSOGA*QGLQGPR, where A * represents alanine with $^{13}$C, $^{15}$N, or D4 (deuterated) labeling, preferably alanine with $^{13}$C, $^{15}$N labeling.

Without binding to any theory, the applicant has determined that the above standard peptide segments are characteristic peptides of type II collagen, and found that by using the above standard peptide segments, especially peptides with the sequence GSOGAQGLQGPR, a method for specifically measuring type II collagen amount can be established. The method has excellent accuracy and sensitivity, and can quickly and effectively identify and quantitatively measure type II collagen.

Furthermore, the applicant found that compared to other amino acid labeling modes, double labeling of glycine in GSOGAQGLQGPR using $^{13}$C and $^{15}$N in the internal standard can significantly improve the accuracy of the detection method.

The recovery rates of GSOGAQGLQGPR peptides labeled with D4 (deuterated) in Quality Control Sample (75ng/mL, 800ng/mL, and 15ug/mL prepared with reference substance) were 90%~125% (test concentration/theoretical concentration).

Table 2-Results of Intra-Ansay Precision Experiment conducted on December 3, 2014.

| 75 ng/mL replicate | calculated concentration (ng/mL) | 800 ng/mL replicate | calculated concentration (ng/mL) | 15 ug/mL replicate | calculated concentration (ng/ml) |
|---|---|---|---|---|---|
| 1 | 76.4 | 1 | 871 | 1 | 14800 |
| 2 | 84.1 | 2 | 857 | 2 | 13700 |
| 3 | 93.0 | 3 | 824 | 3 | 14500 |
| 4 | 77.8 | 4 | 811 | 4 | 14500 |
| 5 | 85.4 | 5 | 629 | 5 | 14400 |
| 6 | 86.2 | 6 | 845 | 6 | 14700 |
| avg | 83.817 | avg | 839.500 | avg | 14433.333 |
| stdev | 6.063 | stdev | 22.323 | stdev | 388.158 |
| %RSD | 7.2 | %RSD | 2.7 | %RSD | 2.7 |

The recovery rate of GSOGAQGLQGPR peptides labeled with $^{13}$C and $^{15}$N in the sample can be found in the working examples.

In some embodiments, the samples are tablets, capsules, granules, powders, small capsules, reconstitutable powders, dry powder inhalers, chewable agents, ointments, pastes, solutions, suspensions, injections, eye drops, liniments, collagen powder or aerosols, preferably tablets. In the specific embodiment, the sample is Vitamins & Minerals type II collagen tablets.

In some embodiments, the type II collagen is derived from human, bovine, mouse, chicken, pig, or monkey sources. In a specific embodiment, the type II collagen comes from chicken cartilage, chicken j oint cartilage, chicken breast cartilage, or pig j oint cartilage.

**Liquid chromatography-tandem mass spectrometry**

In some embodiments, liquid chromatography-tandem mass spectrometry is used to measure the amount of type II collagen in the sample. In a specific embodiment, ultra high performance liquid chromatography-tandem mass spectrometry (UPLC-MS/MS) is used to measure the amount of type II collagen in the sample.

In some embodiments, the ion source of the liquid chromatography-tandem mass spectrometry is selected from electric spray ion source (ESI), atmospheric pressure chemical ionization source (APCI), atmospheric pressure photoionization source (APPI) or matrix assisted laser desorption ionization source (MALDI); The mass analyzer of the liquid chromatography-tandem mass spectrometry is selected from quadrupole mass spectrometry, ion trap mass spectrometry, time of flight (TOF) mass spectrometry, or Fourier transform mass spectrometry. Preferably, the mass spectrometry in the liquid chromatography-tandem mass spectrometry is triple quadrupole mass spectrometry. In a specific embodiment, Waters Xevo TQD tandem mass spectrometer is used.

In liquid chromatography-tandem mass spectrometry, liquid chromatography is used for separating the analyte and interfering substances, while mass spectrometry is used for detection. After the sample is injected, it is first carried by the mobile phase and enters the chromatographic column. After separation by the chromatographic column, it enters the mass spectrometry for detection. Mass spectrometry detects the mass to charge ratio (m/z) of the analyzed substance, which is converted into gas phase ions at the ion source and enters the mass spectrometry. In the triple quadrupole, the primary mass spectrometry scans for specific range of ions or allows specific ions to enter the collision chamber. In the collision chamber, molecular ions collide and decompose, forming sub ions that enter the secondary mass spectrometry. The secondary mass spectrometry scans for specific range of ions or allows specific ions to enter the detector.

In some embodiments, when the standard peptide segment is a peptide with the sequence GSOGAQGLQGPR and the internal standard is a peptide with the sequence GSOG (A-$^{13}$C$^{15}$N) QGLQGPR, the mass to charge ratio for quantifying the characteristic ions of the standard peptide segment is 571.5 and/or 627.4, and the mass to charge ratio of the characteristic ions of the internal standard are 573.5 and/or 627.4. When the standard peptide segment is a peptide with the sequence GSOGAQGLQGPR and the internal standard is a peptide with the sequence GSOG (D4-A) QGLQGPR, the mass to charge ratio of the characteristic ions of the internal standard is 573.5 and/or 887.9.

In a specific embodiment, for the standard peptide segment with the sequence GSOGAQGLQGPR, in a triple quadrupole, primary mass spectrometry scans ions with a mass to charge ratio of 571.5, or allows ions with a mass to charge ratio of 571.5 to enter the collision chamber. Secondary mass spectrometry scans ions with a mass to charge ratio of 627.4 or allows ions with a mass to charge ratio of 627.4 to enter the detector. For internal standards with sequence GSOG (A-$^{13}$C$^{15}$N) QGLQGPR, in a triple quadrupole, primary mass spectrometry scans ions with a mass to charge ratio of 573.5 or allows ions with a mass to charge ratio of 573.5 to enter the collision chamber. Secondary mass spectrometry scans ions with a mass to charge ratio of 627.4 or allows ions with a mass to charge ratio of 627.4 to enter the detector.

In a specific embodiment, solutions containing different concentrations of standard peptide segments and internal standards are prepared for liquid chromatography-tandem mass spectrometry determination. Using the concentration of the standard peptide segment as the x-axis and the peak area ratio of the standard peptide segment to the internal standard as the y-axis, linear simulation is performed to obtain a linear regression curve (i.e. standard curve) and formula. Using the peak area of the standard peptide segment in the sample, the concentration of the standard peptide segment in the sample solution is calculated based on the standard curve, and then the following formula is applied to obtain the amount of type II collagen per unit weight of the sample:

$$\text{Type II Collagen mg/tab} = \frac{Spl.\,Conc. \times DF \times 134999}{1140 \times 1000 \times Wu} \times ATW$$

Wherein:

Spl. Conc. is the concentration of the standard peptide segment in the sample solution ($\mu$g/mL);
DF is the volume or dilution factor of the sample solution (mL);
Wu is the weight of the sample (g);
ATW is the average weight (g) of the sample per unit of formulation, for example, when the sample is a collagen tablet, ATW is the average weight (g) of each tablet;
1140 is the molecular weight of the standard peptide segment;
134999 is the molecular weight of type II collagen.

In a specific embodiment, the peak area of the standard peptide segment is taken as the peak area of the ion with a mass to charge ratio of 627.4, and the peak area of the ion with a mass to charge ratio of 627.4 is taken as the peak area of the internal standard.

In a specific embodiment, the mass spectrometry conditions in the liquid chromatography-tandem mass spectrometry are as follows:

Mass spectrometry: Waters Xevo TQD tandem mass spectrometer
Ion source: ESI, positive ion mode
Scanning method: MS Scan
Detection method: UPLC-MSMS, MRM quantitative
Cone gas: nitrogen

Collision gas: nitrogen

| Source Voltages | Capillary (KV) | 3.72 |
|---|---|---|
| | Cone (V) | 38 |
| Source Temperatures | Desolvation Temp (°C) | 500 |
| Source gas flow | Desolvation (L/Hr) | 900 |
| | Cone (L/Hr) | 10 |
| Analyzer* | LM Resolution 1 | 6.6 |
| | HM Resolution 1 | 13.6 |
| | Ion energy 1 | 0.7 |
| | LM Resolution 2 | 10.0 |
| | HM Resolution 2 | 14.1 |
| | Ion energy 2 | 0.8 |
| | Collision Energy MS (V) | 28 |
| Extended | Source Temp (°C) | 150 |
| | Extractor Voltage (V) | 3 |
| | RF Lens Voltage (V) | 2.5 |
| | Collision Chamber Entrance Voltage (V) | 30 |
| | Collision Chamber Exit Voltage (V) | 30 |
| | Gain | 1 |

In some embodiments, the liquid chromatography in the liquid chromatography-tandem mass spectrometry is reversed phase liquid chromatography, which can use C18, C8, or C4 columns. In a specific embodiment, C18 chromatographic column is used. In a specific embodiment, Waters ACQUITY UPLC BEH C18 chromatographic column is used. In a specific embodiment, a Waters ACQUITY UPLC BEH C18 chromatographic column with a specification of 1.7 $\mu$m and 2.1x50 mm is used.

In some embodiments, the mobile phase used for gradient elution is a mixed system formed by formic acid aqueous solution and water-soluble non-proton organic solvent. The water-soluble non-proton organic solvent is preferably selected from acetonitrile, tetrahydrofuran, and/or dimethylformamide, more preferably acetonitrile.

In some embodiments, the mobile phase comprises mobile phase A and mobile phase B. The mobile phase A is a formic acid aqueous solution, and the mobile phase B is a mixed system of formic acid aqueous solution and water-soluble non-proton organic solvent. In a specific embodiment, the mobile phase A is a formic acid aqueous solution, and the mobile phase B is a mixed system of formic acid aqueous solution and acetonitrile.

In a specific embodiment, the concentration of formic acid in mobile phase A ranges from about 0.001 to 1 vol%, preferably from about 0.01 to 0.5 vol%, more preferably from about 0.05 to 0.5 vol%, such as about 0.05 vol%, about 0.075 vol%, about 0.1 vol%, about 0.2 vol%, or 0.5 vol%, most preferably about 0.1 vol%.

In a specific embodiment, the concentration of formic acid in mobile phase B ranges from about 0.001 to 1 vol%, preferably from about 0.01 to 0.5 vol%, more preferably from about 0.05 to 0.5 vol%, such as about 0.05 vol%, about 0.075 vol%, about 0.1 vol%, about 0.2 vol%, or about 0.5 vol%, most preferably about 0.1 vol%.

In a specific embodiment, the mobile phase A is a formic acid aqueous solution with a concentration of 0.1 vol%, and the mobile phase B is a mixed system of formic acid aqueous solution and acetonitrile, wherein the formic acid concentration is 0.1 vol%.

In a specific embodiment, the elution gradient of the reverse phase liquid chromatography is as follows:

| Time (min) | % Mobile Phase A | %Mobile Phase B |
|---|---|---|
| 0 | 95 | 5 |
| 2 | 20 | 80 |
| 3 | 20 | 80 |

(continued)

| Time (min) | % Mobile Phase A | %Mobile Phase B |
|---|---|---|
| 3.1 | 95 | 5 |
| 7 | 95 | 5 |

In some embodiments, the chromatographic conditions of the reverse phase liquid chromatography also include: The flow rate of the mobile phase is about 0.10 to 1.0 mL/min, preferably about 0.15 to 0.80mL/min, more preferably about 0.15 to 0.5 mL/min, such as, about 0.15 mL/min, about 0.2 mL/min, about 0.25 mL/min, about 0.3 mL/min, about 0.35 mL/min, about 0.4 mL/min, about 0.45 mL/min, or about 0.5 mL/min, most preferably about 0.3 mL/min;

The chromatographic column temperature is about 20 °C to 45 °C, preferably about 30 °C to 45 °C, such as about 30 °C, about 35 °C, about 40 °C, or about 45 °C, preferably about 40 °C;

The detection wavelength is about 180nm to 260nm, preferably about 200nm to 240nm, such as about 200nm, about 210nm, about 220nm, about 230nm or about 240nm, most preferably about 220nm;

The injection volume is about 5μL to 100 μL, preferably about 5 μL to 50 μL, such as about 5 μL, about 7.5 μL, about 10 μL, about 20 μL, about 30 μL, about 40 μL or about 50 μL, most preferably about 10 μL.

**Preparation of Injection Solution**

In an aspect, an injection solution for liquid chromatography-tandem mass spectrometry detection is obtained by preparing the sample to be analyzed into a solution.

In some embodiments, the sample to be analyzed is grinded and dissolved in a solvent to obtain a solution. The solvent can be selected from one or more of water, hydrochloric acid solution, acetonitrile, or methanol, preferably hydrochloric acid solution. The concentration of the hydrochloric acid solution may be about 1 to 10 mol/L, preferably about 3 to 8 mol/L, such as about 3 mol/L, about 4 mol/L, about 5 mol/L, about 6 mol/L, about 7 mol/L, or about 8 mol/L, preferably about 6 mol/L.

In some embodiments, the step of dialysis of the sample solution is also included, where the retained molecular weight of the dialysis bag used ranges from about 100 to 1,000,000 Daltons (D), such as about 500 to 500,000 D, about 1,000 to 250,000 D, about 2,000 to 100,000 D, about 8,000 to 50,000 D, about 10,000 to 20,000 D, or about 12,000 to 14,000 D.

In some embodiments, the dialysis is performed in a dialysis buffer. The dialysis buffer is selected from one or more of deionized water, physiological saline, phosphate buffer, Tris buffer, acetate buffer, and HEPES buffer. In some embodiments, the dialysis is carried out for at least 12 hours, such as 12 to 24 hours.

In some embodiments, internal standards are added to the obtained products after dialysis is completed.

In some embodiments, the protein in the sample to be tested is cleaved before injection detection, which can be enzymatic, chemical, and/or thermal, preferably enzymatic and/or chemical.

For enzymatic hydrolysis, the available proteases are selected from one or more of trypsin, chymotrypsin, thermophilic protease, pepsin, chymotrypsin, glutamate protease, lysine protease, aspartate protease, thrombin, and Staphylococcus aureus protease. In the specific embodiment, trypsin is used.

For chemical cleavage, the available chemical cleavage agents are selected from one or more of cyanide bromide, hydroxylamine hydrochloride, N-bromosuccinimide (NBS), and 3-bromo-3-methyl-2-(2-nitrophenylthio)-3H-indole (BNPS). In a specific embodiment, cyanide bromide is used for chemical cleavage.

In some embodiments, the sample to be analyzed is subject to chemical cleavage and enzymatic hydrolysis, wherein the enzymatic hydrolysis occurs simultaneously with the chemical cleavage, before the chemical cleavage, or after the chemical cleavage. In a specific embodiment, the enzymatic hydrolysis is carried out after the chemical cleavage. In the specific embodiment, trypsin is used for enzymatic hydrolysis after chemical cleavage of the sample using cyanide bromide. In a specific embodiment, the chemical cleavage and/or enzymatic hydrolysis are carried out after adding internal standards to the sample to be tested.

In some embodiments, cyanide bromide is added to the sample to a final concentration range of about 1 to 50 mg/mL, such as about 2 to 20 mg/mL, about 4 to 15 mg/mL, about 8 to 12 mg/mL, or about 10 mg/mL.

In some embodiments, trypsin is added to the sample to achieve a final concentration in the range of about 0.1 to 5 μg/mL, such as about 0.2 to 2 μg/mL, about 0.4 to 1 μg/mL, or about 0.6 to 0.8 μg/mL.

In some embodiments, a reductant is added to the sample before detection, which can be selected from one or more of dithiothreitol (DTT), β-mercaptoethanol, and/or tri(2-carboxyethyl) phosphine (TcEP). In a specific embodiment, the reductant is DTT. In a specific embodiment, DTT is added to the sample to achieve a final concentration within the range of about 1 to 10 mM, such as about 2 to 5 mM or about 3 to 4 mM.

In a specific embodiment, a reductant is added to the sample after the chemical cleavage and before the enzymatic hydrolysis.

In a specific embodiment, samples for liquid chromatography-tandem mass spectrometry detection are prepared by the following steps:

- Grinding the sample to be analyzed and dissolving it in 10 mL of 6 mol/L hydrochloric acid solution to obtain a solution;
- Dialyzing the solution obtained from the above step, with a retaining molecular weight of 12,000 to 14,000 D of the dialysis bag;
- Lyophilizing the solution obtained after dialysis, dissolving the lyophilized product together with the internal standard into 70 vol% formic acid solution, such that the final concentration of the sample to be analyzed is 1 mg/mL, and the final concentration of the internal standard is 500 ng/mL;
- Adding cyanide bromide to the solution obtained from the above step, such that the final concentration of cyanide bromide is 10 mg/mL, shaking the obtained solution overnight in dark, and then lyophilizing to remove cyanide bromide;
- Suspending the dried powder obtained from the above step in chromatography-grade water at a concentration of 1 mg/mL, adding DTT, and incubating at 70 °C for 20 minutes with a final concentration within the range of 3 to 4 mM;
- Cooling the solution obtained from the above step on ice for 10 minutes, then adding trypsin solution to achieve a final concentration of trypsin within the range of 0.6 to 0.8 $\mu$g/mL;
- By adding trifluoroacetic acid (TFA) to terminate the digestion of trypsin, the resulting solution can be injected into liquid chromatography-tandem mass spectrometry.

In a specific embodiment, the sample to be tested is Vitamins & Minerals Type II Collagen Tablets.

**Kit**

In an aspect, provided herein is a kit for quantitative measurement of type II collagen, comprising a standard peptide segment and an internal standard.

In some embodiments, the standard peptide segment is selected from peptides with a sequence GIVGLOGQR or GSOGAQGLQGPR, where O represents hydroxyproline (Hyp). In a specific embodiment, the standard peptide segment is GSOGAQGLQGPR. In a specific embodiment, the standard peptide segment is in the form of a stock solution, with a concentration ranging from about 1 to 100 $\mu$g/mL, such as about 2 to 50 $\mu$g/mL, about 5 to 25 $\mu$g/mL, about 8 to 15 $\mu$g/mL, about 9 to 12 $\mu$g/mL, or about 10 $\mu$g/mL. In a specific embodiment, the solvent in the storage solution can be selected from one or more of deionized water, physiological saline, phosphate buffer, Tris buffer, acetate buffer, and HEPES buffer.

In a specific embodiment, when the standard peptide segment is a peptide with the sequence GSOGAQGLQGPR, the internal standard is a peptide with the sequence GSOGA*QGLQGPR, where A* represents alanine with $^{13}$C, $^{15}$N, or D4 (deuterated) labeling, preferably with $^{13}$C, $^{15}$N labeling. In a specific embodiment, the internal standard is in the form of a stock solution, with concentrations ranging from about 1 to 100 $\mu$g/mL, such as about 2 to 50 $\mu$g/mL, about 5 to 25 $\mu$g/mL, about 8 to 15 $\mu$g/mL, about 9 to 12 $\mu$g/mL, or about 10 $\mu$g/mL. In a specific embodiment, the solvent in the storage solution can be selected from one or more of deionized water, physiological saline, phosphate buffer, Tris buffer, acetate buffer, and HEPES buffer.

In some embodiments, the kit further comprises a protein lysis agent, which can be a protease and/or a chemical cleavage agent. The protease can be selected from one or more of trypsin, chymotrypsin, thermophilic protease, pepsin, chymotrypsin, glutamic acid protease, lysine protease, aspartic acid protease, thrombin, and/or Staphylococcus aureus protease, and the chemical cleaver can be selected from one or more of cyanide bromide, hydroxylamine hydrochloride, N-bromosuccinimide (NBS) and/or 3-bromo-3-methyl-2-(2-nitrophenylthio)-3H-indole (BNPS).

In a specific embodiment, the kit comprises trypsin and cyanide bromide.

In some embodiments, the kit further comprises a reductant. The reductant can be selected from one or more of dithiothreitol (DTT), $\beta$-mercaptoethanol, and/or tris(2-carboxyethyl) phosphine (TcEP). In a specific embodiment, the reductant is DTT.

**EXAMPLES**

In order to better understand the invention described herein, the following embodiments are elaborated. The examples described herein are provided to illustrate the methods provided herein, and are not interpreted in any way to limit their scope.

**Example 1: Preparation of solution**

6N HCl: Measure 247 mL of concentrated hydrochloric acid and dilute to 500 mL with water.

70 vol% formic acid: Measure 140mL of formic acid and dilute to 200mL with water.

5% $Na_2S_2O_3$: Weigh approximately 157.0 g of $Na_2S_2O_3 \cdot 5H_2O$ and dissolve it in water to a volume of 2000 mL.

10 vol% trifluoroacetic acid (TFA): Measure 1.0 mL of TFA and dilute to 10 mL with water.

1M Tris buffer: Weigh about 12.1 g of Tris base and dissolve it in 80 mL water. Adjust the pH to 7.6 using a 6N hydrochloric acid solution, and finally bring to 100 mL.

100 mM CaCh: Weigh 110.1 mg of CaCl2 and dissolve it in water to a volume of 10 mL. Prepare on-site before use.

Trypsin diluent (50 mM Tris buffer, pH 7.6, containing 1 mM CaCh): Measure 100 μL of 100 mM CaCh and 500 mL of 1 M Tris buffer, and dilute to 10 mL with water. Prepare on-site before use.

Trypsin solution: Measure 200 μL of 50 mM acetic acid solution and add it to a trypsin tube (20 μg/rod). Accurately measure 100 mL of the above solution and dilute to 10 mL with trypsin diluent. Prepare on-site before use.

100 mM DTT: Weigh 15.0 mg of DTT and dissolve it in 1 mL of water. Prepare on-site before use.

Mobile phase A: Measure 2.0 mL of 50 vol% formic acid and dilute to 1000 mL with water.

Mobile phase B: Measure 2.0 mL of 50 vol% formic acid and dilute to 1000 mL with acetonitrile.

## Example 2: Establishing a standard curve

Use peptides with the following sequences as standard peptide segments and internal standards:

Table 1:

| Name | Purity | Sequence | Molecular weight |
|---|---|---|---|
| Reference standard peptide segment 1 | >95% | GSOGAQGLQGPR | 1140 |
| Internal standard peptide segment | >95% | GSOGA*QGLQGPR | 1144 |

Wherein, O represents hydroxyproline (Hyp), and A* represents alanine with $^{13}$C and $^{15}$N labeling.

Table 2: Characteristic Ions of Standard Peptide Segment 1 and Internal Standard

| | Sequence | Selected characteristic ions through primary mass spectrometry | Detected fragment ions |
|---|---|---|---|
| Reference peptide segment 1 | GSOGAQGLQGPR | M/z 571.5 | M/z 627.4 |
| Internal standard peptide segment | GSOGA*QGLQGPR | M/z 573.5 | M/z 627.4 |

Reference standard peptide 1 stock solution

Weigh 10.0 mg of standard peptide segment 1 and dilute to 100 mL with water. Measure 10.0 mL of the obtained solution and dilute to 100 mL with water to obtain a standard peptide segment 1 stock solution with a concentration of 10 μg/mL.

Prepare the control peptide 1 stock solution using the same method in the same way.

Internal standard stock solution

Weigh 10.0 mg of internal standard and dilute to 100 mL with water. Measure 10.0 mL of the obtained solution and dilute to 100 mL with water to obtain an internal standard stock solution with a concentration of 10 μg/mL.

Working standard solution

Measure the corresponding volumes of standard peptide segment 1 stock solution and internal standard peptide segment stock solution according to the table below, and dilute to 100 mL with water.

Table 3:

| | Volume of reference standard peptide 1 stock solution | Volume of internal standard stock solution |
|---|---|---|
| STD1 | 1.0 mL | 1.0 mL |
| STD2 | 2.5 mL | 1.0 mL |

(continued)

|  | Volume of reference standard peptide 1 stock solution | Volume of internal standard stock solution |
|---|---|---|
| STD3 | 5.0 mL | 1.0 mL |
| STD4 | 7.5 mL | 1.0 mL |
| STD5 | 10.0 mL | 1.0 mL |

Control reference standard solution

Measure the corresponding volumes of reference standard peptide 1 stock solution and internal standard stock solution according to the table below, and dilute to 100 mL with water.

Table 4:

|  | Volume of reference standard peptide 1 stock solution | Volume of internal standard stock solution |
|---|---|---|
| Control reference standard solution 1 | 2.0 mL | 1.0 mL |
| Control reference standard solution 2 | 4.0 mL | 1.0 mL |
| Control reference standard solution 3 | 8.0 mL | 1.0 mL |

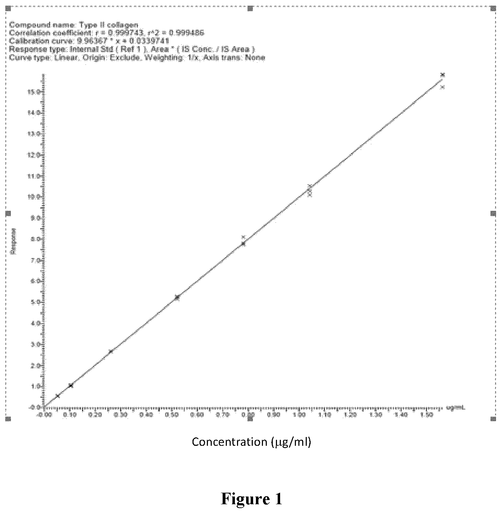

Use liquid chromatography-tandem mass spectrometry to measure the above control reference standard solutions. Linear simulation was performed using the concentration of standard peptide segment 1 as the x-axis and the peak area ratio (relative response) of standard peptide segment 1 to internal standard as the y-axis to obtain the standard curve and formula. The standard curve results are shown in **Figure 1**.

Table 5: Linear curve of control reference substance solution

| Peptide Segment 1 concentration of control reference standard solution (μg/ml) | Relative Response | Summary of Each Concentration | | |
|---|---|---|---|---|
|  |  | Average Relative Response | Standard Deviation | %RSD |
| 0.0521 | 0.5657 | 0.5558 | 0.0086 | 1.54% |
| | 0.5514 | | | |
| | 0.5504 | | | |
| 0.1042 | 1.0512 | 1.0483 | 0.0246 | 2.35% |
| | 1.0223 | | | |
| | 1.0713 | | | |
| 0.2605 | 2.6724 | 2.6767 | 0.0038 | 0.14% |
| | 2.6785 | | | |
| | 2.6793 | | | |
| 0.5210 | 5.2641 | 5.2286 | 0.0669 | 1.28% |
| | 5.2703 | | | |
| | 5.1514 | | | |
| 0.7815 | 8.1085 | 7.8866 | 0.1944 | 2.47% |
| | 7.8049 | | | |
| | 7.7463 | | | |
| 1.0420 | 10.5349 | 10.3080 | 0.2182 | 2.12% |
| | 10.2894 | | | |
| | 10.0997 | | | |

(continued)

| Peptide Segment 1 concentration of control reference standard solution (μg/ml) | Relative Response | Summary of Each Concentration | | |
|---|---|---|---|---|
| | | Average Relative Response | Standard Deviation | %RSD |
| 1.5630 | 15.8285 | 15.6197 | 0.3410 | 2.18% |
| | 15.8043 | | | |
| | 15.2262 | | | |

Table 6: Summary of Linear Results

| Slope | Y-axis intercept | Correlation coefficient (r) | Coefficient of determination ($r^2$) |
|---|---|---|---|
| 9.96367 | 0.03397 | 0.9997 | 0.9995 |

Standard curve linear regression equation:

$$\frac{Peak\ Area\ of\ Control\ Reference\ Standard\ Solution\ (peptide\ segment\ 1)}{Peak\ Area\ of\ Internal\ Standard}$$

$$= 9.96367 * Concentration\ of\ Peptide\ Segment\ 1\ in\ Control\ Reference\ Standard\ Solution$$

$$+ 0.0339741$$

The mass spectrometry and liquid chromatography conditions used are as follows: Mass spectrometry: Waters Xevo TQD tandem mass spectrometer

Ion source: ESI, positive ion mode
Scanning method: MS Scan
Detection method: UPLC-MSMS, MRM quantitative
Cone gas: nitrogen
Collision gas: nitrogen

| | | |
|---|---|---|
| Source Voltages | Capillary (KV) | 3.72 |
| | Cone (V) | 38 |
| Source Temperatures | Desolvation Temp (°C) | 500 |
| Source gas flow | Desolvation (L/Hr) | 900 |
| | Cone (L/Hr) | 10 |
| Analyzer* | LM Resolution 1 | 6.6 |
| | HM Resolution 1 | 13.6 |
| | Ion energy 1 | 0.7 |
| | LM Resolution 2 | 10.0 |
| | HM Resolution 2 | 14.1 |
| | Ion energy 2 | 0.8 |
| | Collision Energy MS (V) | 28 |

(continued)

| | | |
|---|---|---|
| Extended | Source Temp (°C) | 150 |
| | Extractor Voltage (V) | 3 |
| | RF Lens Voltage (V) | 2.5 |
| | Collision Chamber Entrance Voltage (V) | 30 |
| | Collision Chamber Exit Voltage (V) | 30 |
| | Gain | 1 |

UPLC:

Chromatographic column: Waters ACQUITY UPLC BEH C18 1.7mm 2.1 × 50mm
Flow rate: 0.3 ml/min
Injection volume: 10 $\mu$L
Column temperature: 40 °C
Detection wavelength: 220nm
Elution gradient:

| Time (min) | % Mobile Phase A | %Mobile Phase B |
|---|---|---|
| 0 | 95 | 5 |
| 2 | 20 | 80 |
| 3 | 20 | 80 |
| 3.1 | 95 | 5 |
| 7 | 95 | 5 |

MS: MRM, 0min to 7min:

| | Compound name | Parent ion (m/z) | Sub ion (m/z) | Cone voltage (V) | Collision voltage (V) |
|---|---|---|---|---|---|
| 1 | Reference standard peptide segment 1 | 571.5 | 627.4 | 38 | 28 |
| 2 | Internal standard peptide segment | 573.5 | 627.4 | 38 | 28 |

**Example 3: Measuring the amount of type II collagen in Vitamins & Minerals Type II Collagen tablet using liquid chromatography-tandem mass spectrometry**

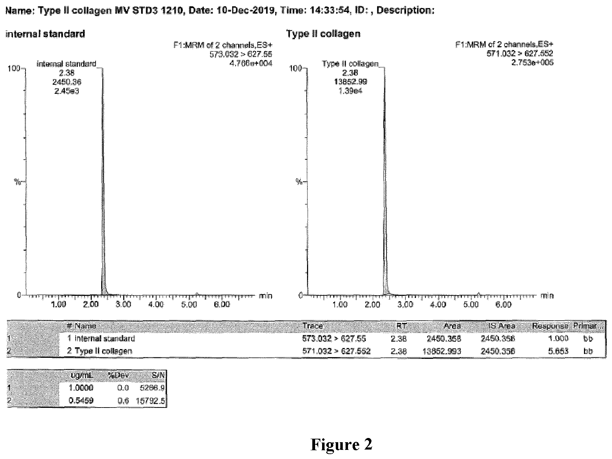

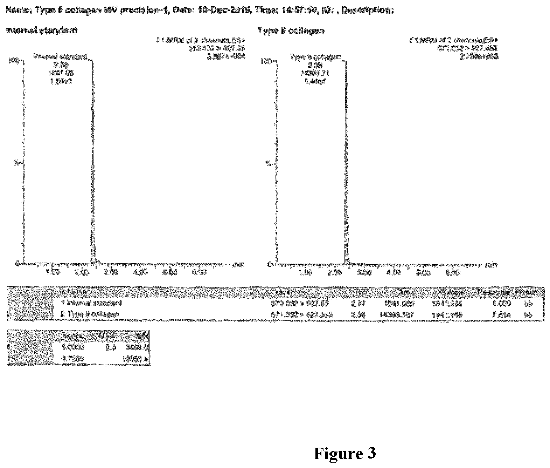

Using a coffee bean grinder to grind 1 tablet of Vitamins & Minerals Type II Collagen and dissolve in 10 mL of 6N hydrochloric acid solution. Transfer the obtained solution to a dialysis bag (with a retained molecular weight of 12,000 to 14,000D). Perform dialysis in at least 250 mL of deionized water. Change the water after 4 hours and repeat 3 times.
After dialysis is completed, transfer the solution in the dialysis bag to a 50 mL polyethylene tube, freeze it in a dry ice/acetone bath, and lyophilize for 24 to 48 hours until completely dry. Weigh the obtained dry powder, add the same internal standard as used in Example 2, and dissolve it in 70 vol% formic acid solution to obtain a solution with a final concentration of 1 mg/mL of the sample to be analyzed and 500 ng/mL of the internal standard.
Measure 1 mL of the above solution and place it in a 5 mL pipette, add 10 mg of cyanide bromide. Seal the pipette and use a swing mixer to stir overnight in dark. Transfer the solution to a 10 mL test tube the next day and lyophilize overnight. Repeat 3 times to lyophilize for at least 24 hours to completely remove cyanide bromide. Resuspend the dried sample in LC MS grade water at a concentration of 1 mg/mL.
Measure 25 $\mu$L of the above solution, place it in a centrifuge tube, and add 5 $\mu$L of 100 mM DTT. Incubate at 70 °C for 20 minutes. Afterwards, cool the solution on ice for 10 minutes. Add 65 $\mu$L of trypsin solution to the above solution and incubate at 37 °C for 18 hours. Afterwards, the reaction was terminated by adding 2 $\mu$L of 10 vol% TFA.
Inject 10 $\mu$L of the obtained solution into liquid chromatography-tandem chromatography. Measure the above solution using the liquid chromatography-tandem mass spectrometry conditions described in Example 2. The chromatograms are shown in **Figures 2 and 3**.

Using the relative response obtained from the ratio of the chromatographic peak area of the reference standard peptide segment in the sample to the internal standard chromatographic peak area, calculate the concentration of the standard peptide segment in the sample solution based on the standard curve, and then use the following formula to obtain the amount of type II collagen per unit weight of the sample:

$$\text{Type II Collagen mg/tab} = \frac{Spl.\,Conc.\times DF \times 134999}{1140 \times 1000 \times Wu} \times ATW$$

Wherein:

Spl. Conc. is the concentration of the standard peptide segment in the sample solution ($\mu$g/mL);

DF is the volume or dilution factor of the sample solution (mL);

Wu is the weight of the sample (g);

ATW is the average weight (g) of the sample per unit of formulation, for example, when the sample is a collagen tablet, ATW is the average weight (g) of each tablet;

1140 is the molecular weight of the standard peptide segment;

134999 is the molecular weight of type II collagen.

Table 7: Recovery results of Type II collagen in Vitamins & Minerals Type II Collagen tablets

| Concentration | Accuracy simulation Weighed Sample g | Theoretical Amount of added Type II Collagen $\mu$g/g | Measured Amount of Type II Collagen $\mu$g/g | Recovery % | Summary of Each Concentration | | |
| --- | --- | --- | --- | --- | --- | --- | --- |
| | | | | | Average Recovery% | Standard Deviation | %RSD |
| 80% | 1.4969 | 6.5415 | 7.1326 | 109.04% | 101.80% | 0.06 | 6% |
| 80% | 1.5023 | 6.5415 | 6.4495 | 98.59% | | | |
| 80% | 1.5125 | 6.5415 | 6.3951 | 97.76% | | | |
| 100% | 1.5725 | 8.3785 | 8.8139 | 105.20% | 104.86% | 0.05 | 4% |
| 100% | 1.5723 | 8.3785 | 8.3918 | 100.16% | | | |
| 100% | 1.5686 | 8.3785 | 9.1514 | 109.22% | | | |
| 150% | 1.5269 | 12.6036 | 13.7321 | 108.95% | 98.57% | 0.10 | 10% |
| 150% | 1.5668 | 12.6036 | 12.3635 | 98.10% | | | |
| 150% | 1.5519 | 12.6036 | 11.1728 | 88.65% | | | |

Table 8: Accuracy for 10 reference standard solution STD1:

| No. | Relative Response* |
| --- | --- |
| 1 | 5.1407 |
| 2 | 5.2631 |
| 3 | 5.1246 |
| 4 | 5.2788 |
| 5 | 5.2280 |
| 6 | 5.3137 |

(continued)

| No. | Relative Response* |
|---|---|
| 7 | 5.3487 |
| 8 | 5.1293 |
| 9 | 5.2278 |
| 10 | 5.2921 |
| Average | 5.23 |
| %RSD | 1.5% |
| *Relative response=peak area of peptide segment 1 in the standard reference solution/peak area of internal standard peptide segment in the standard reference solution | |

Table 9: Study of Accuracy of the Measurement of the Amount of Type II collagen in Vitamins & Minerals Type II Collagen Tablets:

| | Lab 1 | Lab 2 |
|---|---|---|
| Result (mg/tablet) | 17.58 | 16.92 |
| | 18.05 | 16.78 |
| | 16.81 | 17.12 |
| | 17.36 | 17.06 |
| | 17.66 | 16.95 |
| | 17.87 | 16.74 |
| Average (mg/tablet) | 17.55 | 16.93 |
| RSD% | 2.49% | 0.88% |
| Total Average (mg/tablet) | 17.24 | |
| Total RSD% | 2.6% | |
| %Difference | 3.6% | |

**Equivalents**

The present invention can be implemented in other specific forms without departing from its spirit or basic features. Therefore, the aforementioned embodiment should be considered exemplary in all aspects rather than limiting the present invention as described herein. Therefore, the scope of the present invention is indicated by the accompanying claims rather than a specific example mentioned above, and all changes falling within the meaning and scope of the equivalents of the claims are intended to be covered therein.

**Claims**

1. A measurement method for type II collagen, comprising the following steps:

   (1) obtaining a solution of a sample;
   (2) measuring the amount of type II collagen in the solution obtained from step (1) using liquid chromatography-tandem mass spectrometry, preferably using ultra high performance liquid chromatography-tandem mass spectrometry to measure the amount of type II collagen in the solution obtained from step (1).

2. The method as claimed in claim 1, wherein step (2) comprises: (i) measuring the amount of a standard peptide segment of type II collagen in the solution obtained from step (1) using liquid chromatography-tandem mass spectrometry; and (ii) converting the amount of the standard peptide segment measured in step (i) to the amount of type II collagen, wherein the standard peptide segment is selected from peptides with the sequence GIVGLOGQR or GSOGAQGLQGPR, preferably GSOGAQGLQGPR, where O represents hydroxyproline (Hyp).

3. The method as claimed in claim 2, wherein the amount of the standard peptide segment is measured using an internal standard method; when the standard peptide segment is the peptide with sequence GSOGAQGLQGPR, the internal standard is preferably a peptide with sequence GSOGA*QGLQGPR, where A* represents alanine with $^{13}$C, $^{15}$N, or D4 (deuterated) labeling, preferably alanine with $^{13}$C, $^{15}$N labeling.

4. The method as claimed in claim 3, wherein when the standard peptide segment is the peptide with the sequence GSOGAQGLQGPR and the internal standard is the peptide with the sequence GSOG (A-$^{13}$C$^{15}$N) QGLQGPR, the mass to charge ratio of characteristic ions for quantifying the standard peptide segment is 571.5 and/or 627.4, and the mass to charge ratio of characteristic ions of the internal standard is 573.5 and/or 627.4, preferably, the amount of the standard peptide segment is determined based on the peak area of the characteristic ion with a mass to charge ratio of 627.4; when the standard peptide segment is a peptide with the sequence GSOGAQGLQGPR and the internal standard is a peptide with the sequence GSOG(D4-A)QGLQGPR, the mass to charge ratio of the characteristic ions of the internal standard is 573.5 and/or 887.9.

5. The method according to any one of the preceding claims, wherein the ion source of the mass spectrum in the liquid chromatography-tandem mass spectrometry is selected from electric spray ion source (ESI), atmospheric pressure chemical ionization source (APCI), atmospheric pressure photoionization source (APPI) or matrix assisted laser desorption ionization source (MALDI), preferably ESI in positive ion mode; the quality analyzer is selected from quadrupole mass spectrometry, ion trap mass spectrometry, time of flight (TOF) mass spectrometry, or Fourier transform mass spectrometry; preferably, the mass spectrometry in the liquid chromatography-tandem mass spectrometry is a triple quadrupole mass spectrometry.

6. The method as claimed in claim 5, wherein the liquid chromatography is a reversed phase high-performance liquid chromatography, preferably a C18 column, C8 column, or C4 column, and more preferably using a C18 column.

7. The method as claimed in claim 6, wherein gradient elution is performed on a mobile phase, wherein the mobile phase is a mixed system formed by a formic acid aqueous solution and a water-soluble non-proton organic solvent, the water-soluble non-proton organic solvent is preferably selected from acetonitrile, tetrahydrofuran, and/or dimethylformamide, more preferably acetonitrile.

8. The method as claimed in claim 7, wherein the mobile phase comprises a mobile phase A and a mobile phase B, the mobile phase A is a formic acid aqueous solution, and the mobile phase B is a mixed system of formic acid aqueous solution and the water-soluble non-proton organic solvent.

9. The method as claimed in any one of the preceding claims, further comprising the step of protein cleavage in the sample before detection, wherein the cleavage is enzymatic, chemical, and/or thermal, preferably enzymatic and/or chemical,

   when the cleavage is enzymatic hydrolysis, it is preferable to use trypsin, chymotrypsin, thermophilic protease, pepsin, chymotrypsin, glutamic acid protease, lysine protease, aspartic acid protease, thrombin, and/or Staphylococcus aureus protease for enzymatic hydrolysis, and more preferably to use trypsin;
   When the cleavage is chemical cleavage, it is preferable to use cyanide bromide, hydroxylamine hydrochloride, N-bromosuccinimide (NBS) and/or 3-bromo-3-methyl-2-(2-nitrophenylthio)-3H-indole (BNPS), and more preferably to use cyanide bromide;
   Preferably, the sample is subjected to chemical cleavage and enzymatic hydrolysis, wherein the enzymatic hydrolysis is carried out simultaneously with, before, or after the chemical cleavage; more preferably, the enzymatic hydrolysis is performed after the chemical cleavage; further more preferably, trypsin is used for enzymatic hydrolysis after chemical cleavage of the sample using cyanide bromide.

10. The method as claimed in claim 9, further comprising the step of adding a reductant to the sample before detection, wherein the reductant is preferably selected from dithiothreitol (DTT), β-mercaptoethanol, and/or tri(2-carboxyethyl) phosphine (TcEP), more preferably dithiothreitol (DTT); preferably, a reductant is added to the sample after the chemical cleavage and before the enzymatic hydrolysis.

11. The method as claimed in any one of the preceding claims, wherein the sample is a tablet, capsule, granule, powder, small capsule, reconstitutable powder, dry powder inhaler, chewable agent, ointment, paste, solution, suspension, injection, eye drop, liniment, collagen protein powder or aerosol, preferably a tablet.

12. The method according to any one of the preceding claims, wherein the type II collagen is derived from human, bovine, mouse, chicken, pig, or monkey sources, preferably from chicken cartilage, chicken j oint cartilage, chicken chest cartilage, or pig j oint cartilage.

13. A kit, comprising a standard peptide segment and an internal standard.

14. The kit as claimed in claim 13, wherein the standard peptide segment is selected from peptides with a sequence GIVGLOGQR or GSOGAQGLQGPR, preferably GSOGAQGLQGPR, wherein O represents hydroxyproline (Hyp).

15. The kit as claimed in claim 14, when the standard peptide segment is a peptide with sequence GSOGAQGLQGPR, the internal standard is preferably a peptide with the sequence GSOGA*QGLQGPR, where A* represents alanine with $^{13}$C, $^{15}$N, or D4 (deuterated) labeling, preferably alanine with $^{13}$C, $^{15}$N labeling.

16. The kit as claimed in any one of claims 13-15, further comprising a protein lysis agent, wherein the protein lysis agent is a protease and/or a chemical cleavage agent, wherein the protease is preferably selected from trypsin, chymotrypsin, thermophilic protease, pepsin, chymotrypsin, glutaminase, lysine protease, aspartate protease, thrombin, and/or Staphylococcus aureus protease; the chemical cleavage agent is preferably selected from cyanide bromide, hydroxylamine hydrochloride, N-bromosuccinimide (NBS) and/or 3-bromo-3-methyl-2-(2-nitrophenylthio)-3H-indole (BNPS), and more preferably, the kit comprises trypsin and cyanide bromide.

17. The kit as claimed in any one of claims 13-16, further comprising a reductant, which is preferably selected from dithiothreitol (DTT), β-mercaptoethanol, and/or tri(2-carboxyethyl) phosphine (TcEP), more preferably dithiothreitol (DTT),

18. The kit as claimed in any one of claims 13-17 for measuring the amount of type II collagen, preferably for measuring the amount of type II collagen by liquid chromatography-tandem mass spectrometry.

19. Use of peptides selected from the following sequences for measuring the amount of type II collagen: GIVGLOGQR or GSOGAQGLQGPR, preferably GSOGAQGLQGPR, where O represents hydroxyproline (Hyp).

20. The use as claimed in claim 19, wherein the peptide is a peptide with the sequence GSOGA*QGLQGPR, wherein A* represents alanine with $^{13}$C, $^{15}$N, or D4 (deuterated) labeling, preferably alanine with $^{13}$C, $^{15}$N labeling.

21. The use as claimed in claim 20, wherein the peptide is used as an internal standard.

Concentration (µg/ml)

**Figure 1**

Name: Type II collagen MV STD3 1210, Date: 10-Dec-2019, Time: 14:33:54, ID: , Description:

**Figure 2**

Name: Type II collagen MV precision-1, Date: 10-Dec-2019, Time: 14:57:50, ID: , Description:

**Figure 3**

Europäisches
Patentamt

European
Patent Office

Office européen
des brevets

# EUROPEAN SEARCH REPORT

Application Number

EP 23 21 0530

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BENJAMIN J. BIELAJEW: "Methodology to Quantify Collagen Subtypes and Crosslinks: Application in Minipig Cartilages", CARTILAGE, vol. 13, no. 2_suppl, 1 December 2021 (2021-12-01), pages 1742S-1754S, XP093154237, ISSN: 1947-6035, DOI: 10.1177/19476035211060508 | 1,2, 5-14, 16-19 | INV. G01N33/68 C07K14/78 |
| Y | * abstract; page 1743S, right column, line 1 – page 1744S, left-column, line 2; section "sample preparation" on page 1745S; table 1 * | 8,10,17 | |
| X | PRITI PRASANNA MAITY: "Isolation and mass spectrometry based hydroxyproline mapping of type II collagen derived from Capra hircus ear cartilage", COMMUNICATIONS BIOLOGY, vol. 2, no. 1, 29 April 2019 (2019-04-29), XP093154477, ISSN: 2399-3642, DOI: 10.1038/s42003-019-0394-6 Retrieved from the Internet: URL:https://www.nature.com/articles/s42003 -019-0394-6> * abstract; table 1; page 8, last par. – page 9, 2nd par. * | 1,2,10, 19 | |
| X | JP 2016 028100 A (NIPPI INC) 25 February 2016 (2016-02-25) | 1-7,9, 11-16, 18-21 | |
| Y | * the whole document, especially par. [0014], [0015], [0023], [0024], [0040]-[0046], [0123], [0131], [0138] * | 8,10,17 | |

TECHNICAL FIELDS SEARCHED (IPC)

G01N
C07K

The present search report has been drawn up for all claims

| Place of search | Date of completion of the search | Examiner |
|---|---|---|
| Munich | 1 May 2024 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 23 21 0530

This annex lists the patent family members relating to the patent documents cited in the above-mentioned European search report.
The members are as contained in the European Patent Office EDP file on
The European Patent Office is in no way liable for these particulars which are merely given for the purpose of information.

01-05-2024

| Patent document cited in search report | Publication date | Patent family member(s) | Publication date |
|---|---|---|---|
| JP 2016028100 A | 25-02-2016 | JP 5845236 B2 | 20-01-2016 |
| | | JP 6301298 B2 | 28-03-2018 |
| | | JP 2014130134 A | 10-07-2014 |
| | | JP 2016028100 A | 25-02-2016 |

EPO FORM P0459

For more details about this annex : see Official Journal of the European Patent Office, No. 12/82